# EUROPÄISCHE PATENTSCHRIFT

(11) **EP 0 758 322 B1**
(45) Veröffentlichungstag und Bekanntmachung des Hinweises auf die Patenterteilung: **05.09.2001**
(21) Anmeldenummer: 95918603.2
(22) Anmeldetag: 25.04.1995
(51) Int. Cl.: C07D 231/20, C07D 231/08, C07D 401/04, C07D 403/04, A01N 43/56

(54) **ALPHA-(PYRAZOL-3-YL)-OXYMETHYLEN-PHENYLBUTENSÄUREMETHYLESTER UND IHRE VERWENDUNG ZUR BEKÄMPFUNG VON SCHADPILZEN ODER TIERISCHEN SCHÄDLINGEN**
ALPHA-(PYRAZOL-3-YL)-OXYMETHYLENE-PHENYLBUTENIC ACID METHYL ESTERS AND THE USE THEREOF AGAINST HARMFUL FUNGI OR ANIMAL PESTS
METHYLESTERS D'ACIDE ALPHA-(PYRAZOL-3-YL)-OXYMETHYLENE-PHENYLBUTENIQUE ET LEUR UTILISATION CONTRE DES CHAMPIGNONS OU DES ANIMAUX NUISIBLES

(30) Priorität: 03.05.1994 DE 4415483
(43) Veröffentlichungstag der Anmeldung: 19.02.1997
(73) Patentinhaber: BASF AKTIENGESELLSCHAFT, 67056 Ludwigshafen (DE)
(72) Erfinder: OBERDORF, Klaus, D-69117 Heidelberg (DE); KÖNIG, Hartmann, D-69115 Heidelberg (DE); MÜLLER, Bernd, D-67227 Frankenthal (DE); KIRSTGEN, Reinhard, D-67434 Neustadt (DE); GRAMMENOS, Wassilios, D-67063 Ludwigshafen (DE); SAUTER, Hubert, D-68167 Mannheim (DE); LORENZ, Gisela, D-67434 Hambach (DE); AMMERMANN, Eberhard, D-64646 Heppenheim (DE); HARRIES, Volker, D-67227 Frankenthal (DE)
(74) Vertreter: Fitzner, Uwe, Dr.
(86) Internationale Anmeldenummer: EP9501554
(87) Internationale Veröffentlichungsnummer: WO9529896

(56) Entgegenhaltungen:
- EP-A- 0 280 185
- EP-A- 0 513 580

## Beschreibung

Die vorliegende Erfindung betrifft α-Phenylbutensäuremethylester der Formel 1 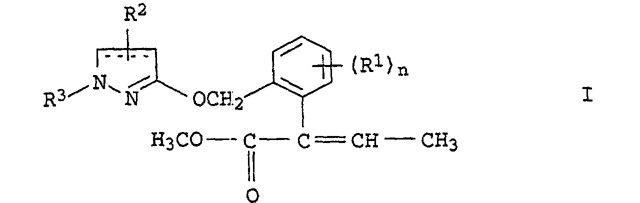 in der -- für eine Einfach- oder Doppelbindung steht und der Index und die Substituenten die folgende Bedeutung haben:
- n: 0, 1, 2, 3 oder 4, wobei die Substituenten R¹ verschieden sein können, wenn n größer als 1 ist;
- R¹: Nitro, Cyano, Halogen, C₁-C₄-Alkyl, C₁-C₄-Halogenalkyl oder C₁-C₄-Alkoxy;
- R²: Wasserstoff, Nitro, Cyano, Halogen, C₁-C₄-Alkyl, C₁-C₄-Halogenalkyl, C₁-C₄-Alkoxy, C₁-C₄-Alkylthio oder C₁-C₄-Alkoxycarbonyl ;
- R³: C₁-C₁₀-Alkyl, C₂-C₁₀-Alkenyl oder C₂-C₂₀-Alkinyl; wobei diese Gruppen partiell oder vollständig halogeniert sein können und/oder einen bis drei der folgenden Reste tragen können:
Cyano, Nitro, C₁-C₄-Alkoxy, C₁-C₄-Halalkoxy, C₁-C₄-Alkylthio oder Phenyl und Naphthyl und 5-Ring Heteroaromaten enthaltend ein bis drei Stickstoffatome und/oder ein Sauerstoff- oder Schwefelatom oder Sechsring Heteroatomaten enthaltend ein bis vier Stickstoffatome,
wobei die cyclischen Gruppen partiell oder vollständig halogeniert sein können und/oder einen bis drei der folgenden Reste tragen können: Nitro, Cyano, C₁-C₄-Alkyl oder C₁-C₄-Alkoxy;
ein gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, oder
ein ein- oder zweikerniger aromatischer Rest, welcher neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann,
wobei die cyclischen Gruppen partiell oder vollständig halogeniert sein können und/oder einen bis drei der folgenden Reste tragen können: Nitro, Cyano, C₁-C₄-Alkyl oder C₁-C₄-Alkoxy.

Außerdem betrifft die Erfindung Verfahren zur Herstellung dieser Verbindungen, sie enthaltende Mischungen sowie ihre Verwendung zur Bekämpfung von Schadpilzen oder tierischen Schädlingen, ausgenommen am tierischen Körper.

Aus der EP-A 513 580 sind α-Phenylbutensäuremethylester, welche in ortho Position einen 4-Pyrazolyloxymethylenrest tragen, mit Wirkung gegen Schadpilze oder tierische Schädlinge bekannt. Die Wirkung dieser Verbindungen ist jedoch bei niedrigeren Aufwandmengen nicht befriedigend.

Der vorliegenden Erfindung lagen daher Verbindungen mit verbessterten Wirkungen als Aufgabe zugrunde.

Demgemäß wurden die eingangs definierten Verbindungen I gefunden. Desweiteren wurden Verfahren zu ihrer Herstellung, sie enthaltende Mischungen sowie Verfahren zur Bekämpfung von Schadpilzen und tierischen Schädlingen unter Verwendung der Verbindungen I gefunden.

Die Verbindungen I sind auf verschiedenen aus der Literatur an sich bekannten Methoden erhältlich.

Beispielsweise erhält man die Verbindungen I durch Umsetzung des Benzylderivats der Formel. II mit einem 3-Hydroxy(dihydro)pyrazol der Formel III in Gegenwart einer Base.

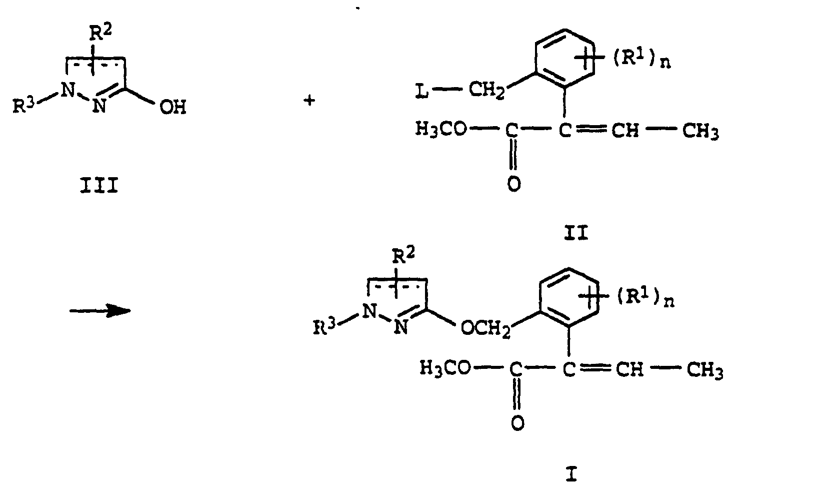

L in der Formel II bedeutet eine nucleophil austauschbare Gruppe, beispielsweise Halogen, z.B. Chlor, Brom und Iod, oder ein Alkyl- oder Arylsulfonat, z.B. Methylsulfonat, Trifluormethylsulfonat, Phenylsulfonat und 4-Methylphenylsulfonat.

Die Umsetzung wird üblicherweise bei Temperaturen von 0°C bis 80°C, vorzugsweise 20°C bis 60°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Dieethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton und Dimethylformamid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium. Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.

Es kann für die Umsetzung vorteilhaft sein, zunächst das 3-Hydroxy(dihydro)pyrazol mit der Base in das entsprechende Hydroxylat umzusetzen, welches dann mit dem Benzylderivat umgesetzt wird.

Die für die Herstellung der Verbindungen I benötigten Ausgangsstoffe II sind aus EP-A 513 580 bekannt. Verbindungen II, in denen L für Chlor oder Brom steht, erhält man demgemäß durch Umsetzung von entsprechenden Ethern (Alkyl- oder Arylether) mit Halogenierungsmitteln [Hal] (z.B. Bortrichlorid, Bromwasserstoff) in einem inerten Lösungsmittel (z.B. halogenierte und/oder aromatische Kohlenwasserstoffe) bei Temperaturen von -30°C bis 40°C. 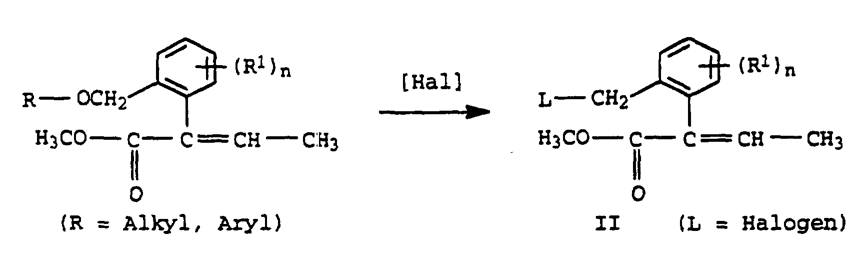 3-Hydroxypyrazole IIIa und 3-Hydroxydihydropyrazole IIIb (bzw. deren tautomere Form: 3-Pyrazolone) sind aus der Literatur bekannt oder können nach den dort beschriebenen Methoden hergestellt werden [IIIa: J. Heterocycl. Chem. 30, 49 (1993); Chem. Ber. 107, 1318 (1974); Chem. Pharm. Bull. 19, 1389 (1971); Tetrahedron Lett. 11, 875 (1970); Chem. Herterocycl. Comp. 5, 527 (1969); Chem. Ber. 102, 3260 (1969); Chem. Ber. 109, 261 (1976); J. Org. Chem. 31, 1538 (1966); Tetrahedron 43, 607 (1987); IIIb: J. Med. Chem. 19, 715 (1976)].

Man erhält die Verbindungen I analog zu den in EP-A 513 580 beschriebenen Methoden nach den im folgenden Reaktionsschema zusammengestellten Verfahren (in Bezug auf die Verfahrensparameter sei auf die Angaben in EP-A 513 580 verwiesen, deren Offenbarung bezüglich der Verfahren zur Herstellung hiermit einbezogen ist).

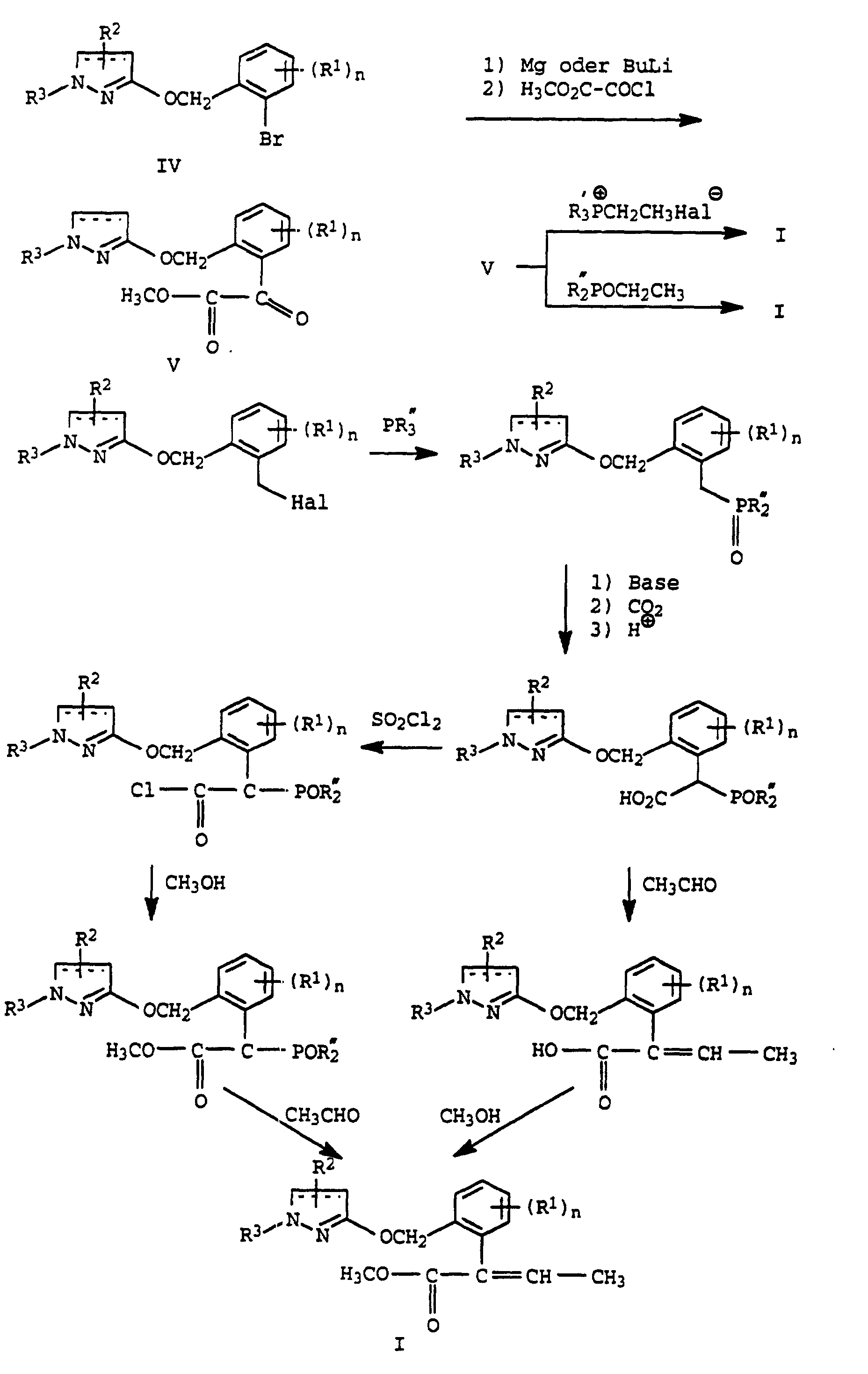

Die Verbindungen I können in Bezug auf die Butensäure-Doppelbindung sowohl als E- als auch als Z-Isomere vorliegen. Beide Isomere können in der erfindungsgemäßen Weise getrennt oder gemeinsam zur Anwendung kommen. Bevorzugt sind Mischungen der Isomere, insbesondere das E-Isomer (Konfiguration bezüglich der Carboxylat-Methyl-Stellung).

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
Alkoxy: geradkettige oder verzweigte Alkylgruppen mit 1 bis 4 Kohlenstoffatomen (wie vorstehend genannt), welche über ein Sauerstoffatom (-O-) an das Gerüst gebunden sind;
Alkoxycarbonyl: geradkettige oder verzweigte Alkoxygruppen mit 1 bis 4 Kohlenstoffatomen (wie vorstehend genannt), welche über eine Carbonylgruppe (-CO-) an das Gerüst gebunden sind;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;
Alkyl: gesättigte, geradkettige oder verzweigte Kohlenwasserstoffreste, insbesondere mit 1 bis 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl und 1-Ethyl-2-methylpropyl;
Alkenyl: ungesättigte, geradkettige oder verzweigte Kohlenwasserstoffreste, insbesondere mit 2 bis 10 Kohlenstoffatomen und einer Doppelbindung in einer beliebigen Position, z.B. C₂-C₆-Alkenyl wie Ethenyl, 1-Propenyl, 2--Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-l-butenyl, 2,3-Dimethyl-2-butenyl, 2,3-Dimethyl-3-butenyl, 3,3-Dimethyl1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, l-Ethyl-1-methyl-2-propenyl, 1-Ethyl-2-methyl-1-propenyl und 1-Ethyl-2-methyl-2-propenyl;
Alkinyl: geradkettige oder verzweigte Kohlenwasserstoffgruppen, insbesondere mit 2 bis 20 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
ein gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl;
oder ein ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazal-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl oder beispielsweise sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5.-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Die cyclischen (gesättigten, ungesättigtern oder aromatischen) Gruppen können partiell oder vollständig halogeniert sein, d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein und/oder einen bis drei, der folgenden Reste tragen: Nitro, Cyano, C₁-C₄-Alkyl oder C₁-C₄-Alkoxy.

Im Hinblick auf ihre biologische Wirkung sind Verbindungen I bevorzugt, in denen n für 0 oder 1 steht.

Gleichermaßen bevorzugt sind Verbindungen I, in denen R¹ für Halogen, C₁-C₄-Alkyl, C₁-C₂-Halogenalkyl und C₁-C₂-Alkoxy steht.

Außerdem werden Verbindungen I bevorzugt, in denen R² für Wasserstoff, Nitro, Halogen, C₁-C₄-Alkyl, C₁-C₂-Halogenalkyl und C₁-C₂-Alkoxycarbonyl steht.

Daneben werden Verbindungen I bevorzugt, in denen R³ für ggf. subst. C₁-C₄-Alkyl oder C₃-C₆-Cycloalkyl steht.

Gleichermaßen werden Verbindungen I bevorzugt, in denen R³ für einen ggf. subst. gesättigten oder ein- oder zweifach ungesättigten Ring steht, welcher neben Kohlenstoffatomen ein bis drei der folgenden Heteroatome als Ringglieder enthalten kann: Sauerstoff, Schwefel und Stickstoff.

Außerdem werden Verbindungen I bevorzugt, in denen R³ für einen ggf. subst. ein- oder zweikernigen aromatischen Rest steht, welcher neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann.

Daneben werden Verbindungen I bevorzugt, in denen R³ für einen ggf. subst. 5-Ring-Heteroaromaten steht.

Desweiteren werden Verbindungen I bevorzugt, in denen R³ für einen ggf. subst. 6-Ring-Heteroaromaten steht.

Insbesondere bevorzugt sind Verbindungen I, in denen R¹ für Wasserstoff (n=0), Chlor, Methyl und Trifluormethyl steht.

Insbesondere sind außerdem Verbindungen I bevorzugt, in denen R² für Wasserstoff, Halogen, Methyl, Trifluormethyl und Methoxycarbonyl steht.

Gleichermaßen werden Verbindungen I insbesondere bevorzugt, in denen R³ für C₁-C₄-Alkyl steht.

Außerdem werden Verbindungen I insbesondere bevorzugt, in denen R³ für C₃-C₆-Cycloalkyl steht, welches einen bis drei C₁-C₄-Alkylgruppen tragen kann.

Insbesondere werden daneben Verbindungen I bevorzugt, in denen R³ für ggf. subst. Phenyl steht.

Insbesondere werden auch Verbindungen I bevorzugt, in denen R³ für ggf. subst. Pyridyl oder Pyrimidyl steht.

Insbesondere sind solche Verbindungen I bevorzugt, in denen R³ für *ggf.* substituiertes Phenyl oder Benzyl steht. Als Substituenten des Phenylrestes kommen in diesen Fällen bevorzugt Halogen, Cyano, C₁-C₄-Alkyl, C₁-C₄-Alkoxy, C₁-C₂-Halogenalkyl, C₁-C₂-Halogenalkoxy, Phenyl und Oxy-C₁-C₂-alkylidenoxy in Betracht.

Ebenfalls bevorzugt sind solche Verbindungen I, in denen R³ für ggf. substituierten Fünfringheteroaromaten wie z.B. Thiazolyl, Isoxazolyl oder Oxazolyl, steht. Als Substituenten des Fünfringheteroaromaten kommen bevorzugt Halogen, Cyano, C₁-C₆-Alkyl, C₁-C₆-Alkoxy, C₁-C₄-Halogenalkyl, C₁-C₂-Halogenalkoxy und Phenyl in Betracht.

Ebenfalls bevorzugt sind solche Verbindungen I, in denen R³ für gegebenenfalls substituierte Sechsringheteroaromaten, wie z.B. Pyridyl, Pyrimidyl, Pyridazinyl oder Pyrazinyl steht. Als Substituenten des Sechsringheteroaromaten kommen bevorzugt Halogen, Cyano, C₁-C₄-Alkyl, C₁-C₄-Alkoxy, C₁-C₂-Halogenalkyl, C₁-C₂-Ralogenalkoxy und Phenyl in Betracht.

Die erfindungsgemäßen α-Phenylbutensäuremethylester der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha., Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis,
ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus,
ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa,
ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria,
ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die α-Phenylbutensäuremethylester der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-Volumeverfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach MMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erf indungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsauremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsaurecyclohexylamid, N-Cyclohexyl-N-methoxy-2,5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorohenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl)-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

### Beispiel 1: α-{2-[1-(4-Chlorphenyl)pyrazol-3-yloxymethyl]phenyl}-but-2-ensäuremethylester

### 1.a α-(2-Brommethylphenyl)but-2-ensäuremethylester

Eine Lösung von 14,8 g α-[2-(2-Methylphenyloxymethyl)phenyl]-but-2-ensäuremethylester in 250 ml Methylenchlorid wurde bei -5°C bis zur Sättigung mit Bromwasserstoff begast (ca. 18 g HBr). Nach Abschluß der Umsetzung (ca. 2 h bei 25°C) wurde das Lösungsmittel bei vermindertem Druck abdestilliert. Der so erhaltene Rückstand wurde in 300 ml Cyclohexan aufgenommen. Die Lösung wurde mit 5 %-iger Natronlauge und anschließend mit Wasser gewaschen. Nach Trocknen und Einengen der organischen Phase erhielt man 8 g der Titelverbindung (Fp: 64-66°C).

### 1.b 1-(4-Chlorphenyl)-3-hydroxypyrazol

Eine Lösung von 57,5 g 4-Chlorphenylhydrazinhydrosulfat in 1000 ml tert.-Butanol wurde zunächst portionsweise mit 100,8 g Kalium-tert.-butylat und anschließend (nach 10 Min. Rühren) innerhalb von 45 Min. bei 45°C-50°C mit einer Lösung aus 27,7 g Propiolsäuremethylester in 90 ml tert.-Butanol versetzt. Nach 1 h bei Siedetemperatur ließ man die Mischung erkalten und entfernte das Lösungsmittel bei vermindertem Druck. Der so erhaltene Rückstand wurde in 1200 ml Wasser gelöst. Die wäßrige Phase wurde zunächst mit Methylenchlorid gewaschen und anschließend angesäuert, wobei das Produkt sich als Feststoff absetzte. Man erhielt 47,6 g der Titelverbindung, Fp: 185-187°C.

### 1.c Eine Mischung aus 2,43 g des Produkts aus 1.b, 2,58 g Kaliumcarbonat, 3,36 g des Produkts aus 1.a und 33 ml Dimethylformamid wurde 4 h bei 60°C gerührt. Nach dem Abkühlen wurde die Reaktionsmischung in 300 ml Wasser aufgenommen. Das Produkt wurde nach Extraktion der wäßrigen Lösung mit Methyl-tert.-butylether aus der organischen Phase isoliert und anschließend chromatographisch gereinigt. (Kieselgel, Cyclohexan/MTBE 3/1). (4,5 g der Titelverbindung, Fp: 65-66°C).

### Beispiel 2: α-{2-[1-phenyl-4,5-dihydropyrazol-3-yloxymethyl]phenyl}-but-2-ensäuremethylester

Eine Mischung aus 2,43 g N-Phenylpyrazolidinon-3, 3,1 g Kaliumcarbonat, 4,04 g des Produkts aus 1.a und 40 ml Dimethylformamid wurde 4 h bei 60°C gerührt. Nach dem Abkühlen wurde die Reaktionsmischung in 300 ml Kochsalzlösung (verdünnt) aufgenommen. Das Produkt wurde nach Extraktion der wäßrigen Lösung mit Methylter.-butylether aus der organischen Phase isoliert und anschließend chromatographisch an Kieselgel mit Cyclohexan/Methyltert.-butylether 5/1 gereinigt (1,4 g der Titelverbindung, Fp: 90-92°C).

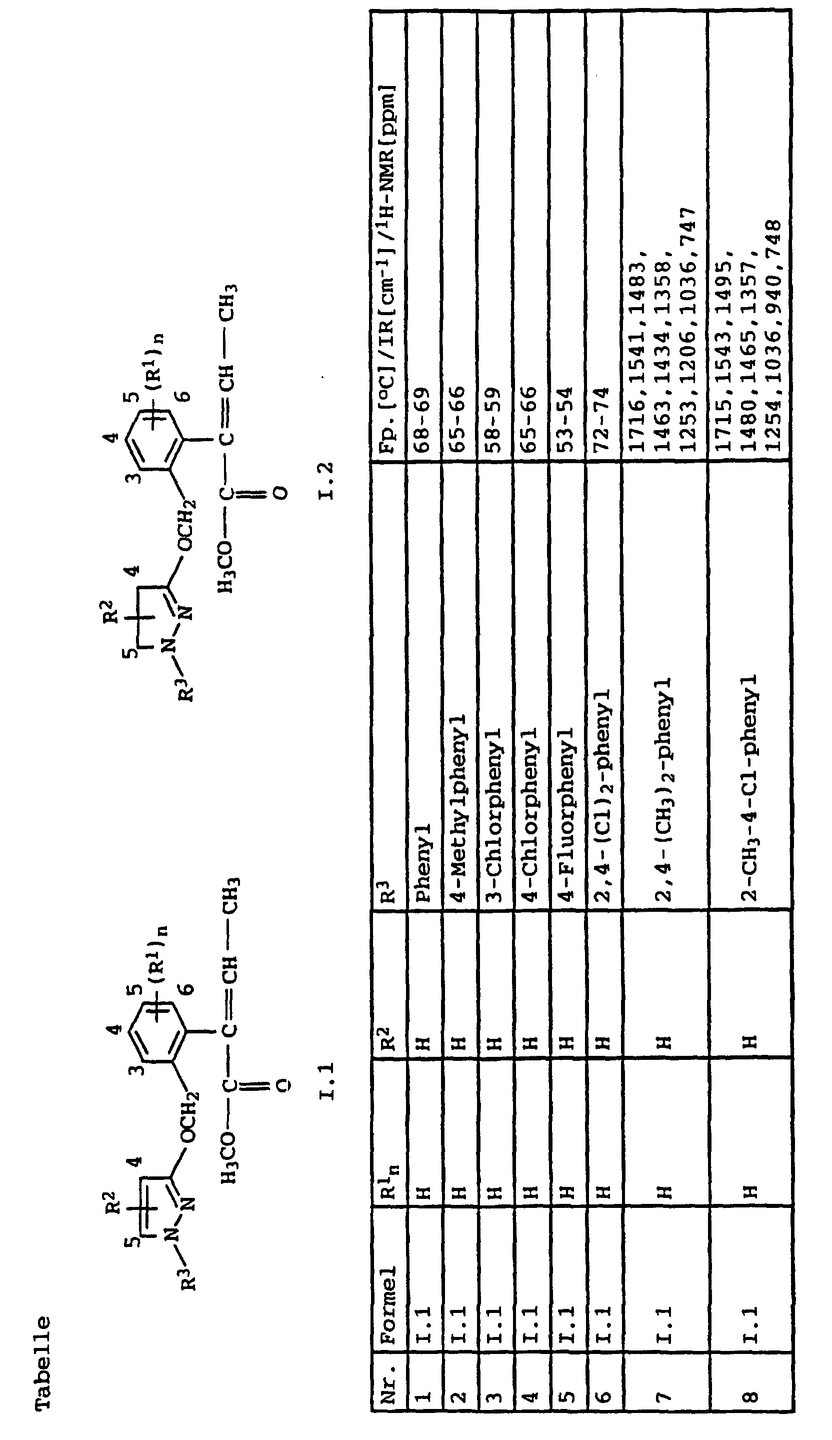

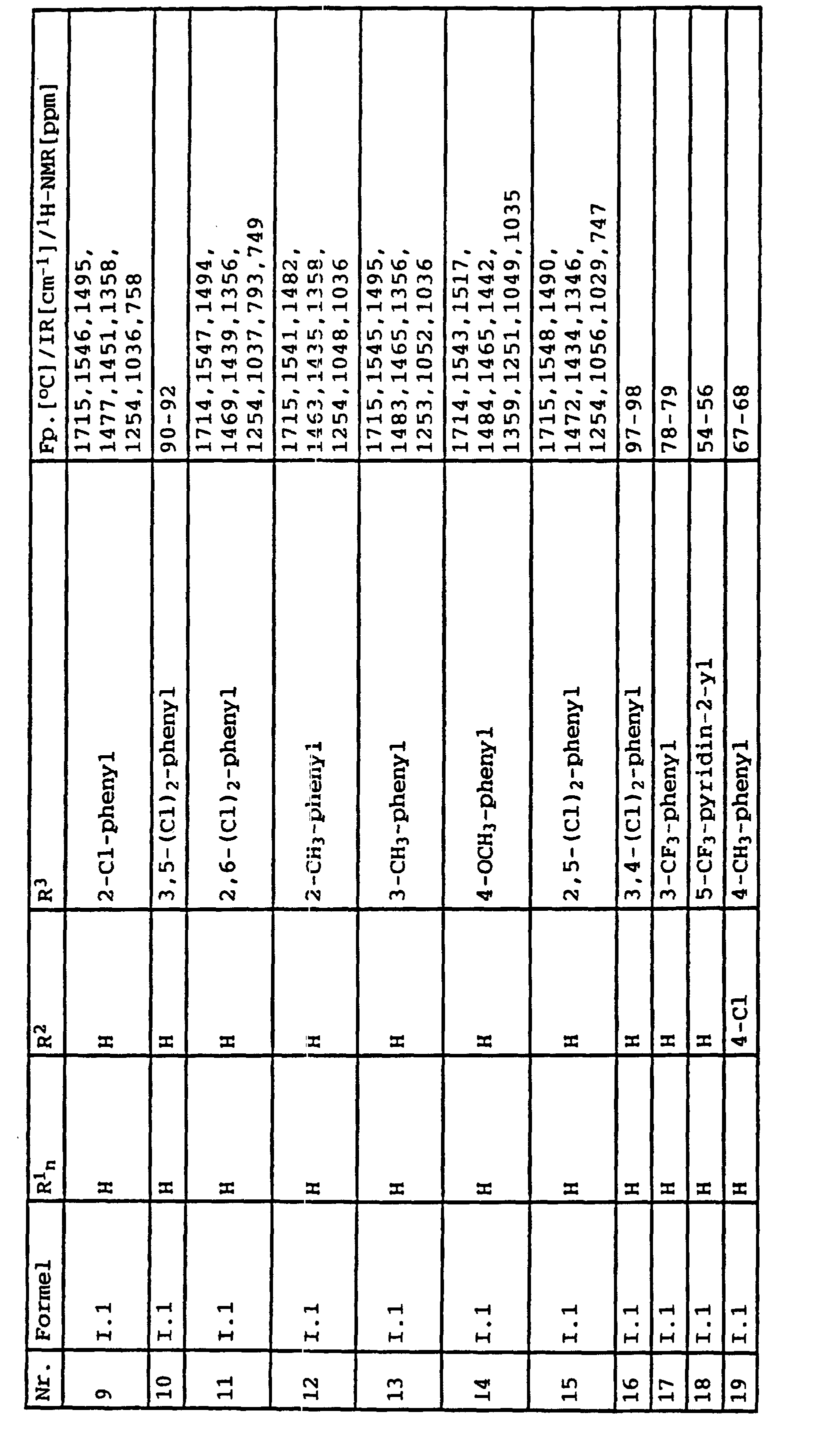

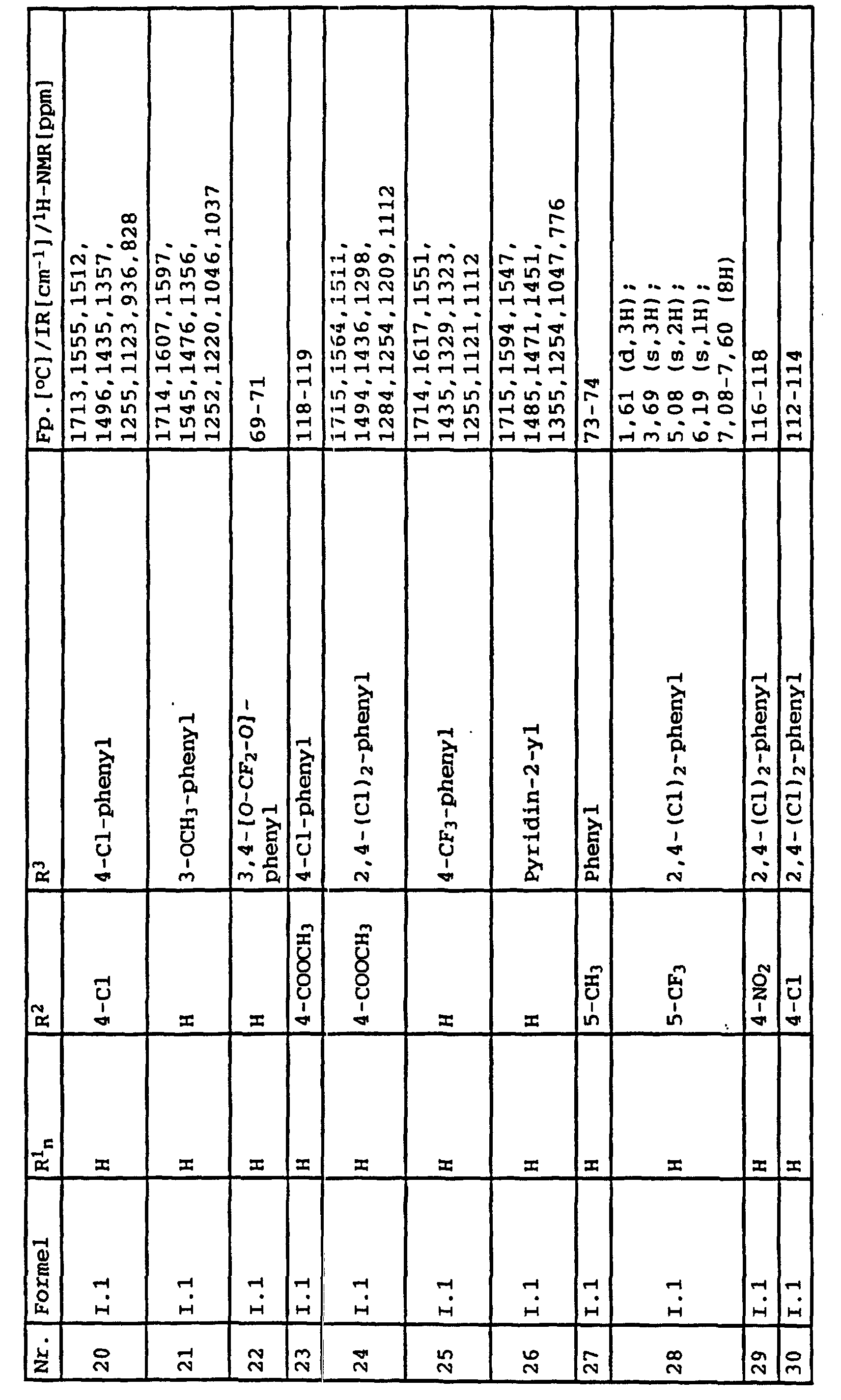

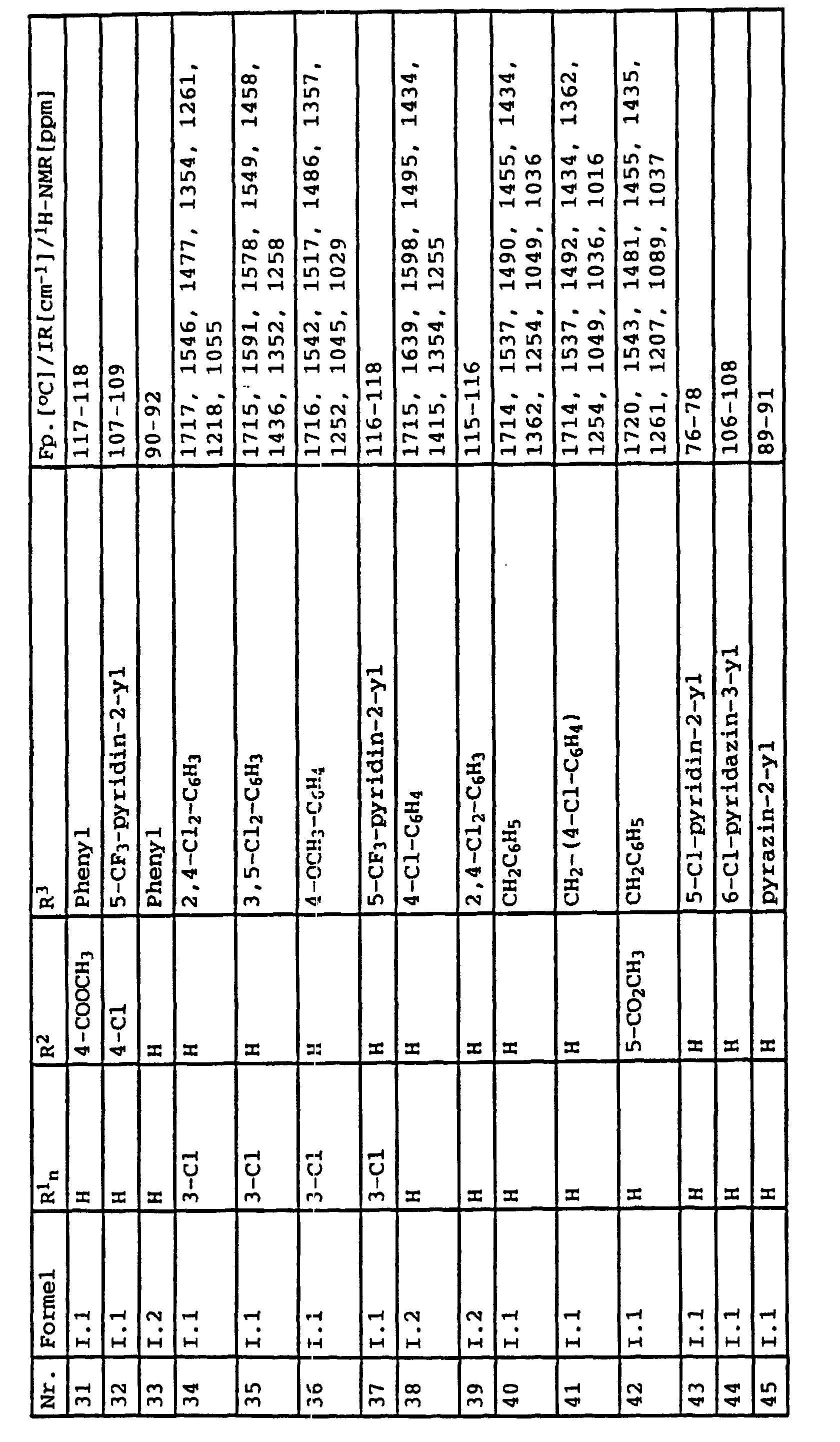

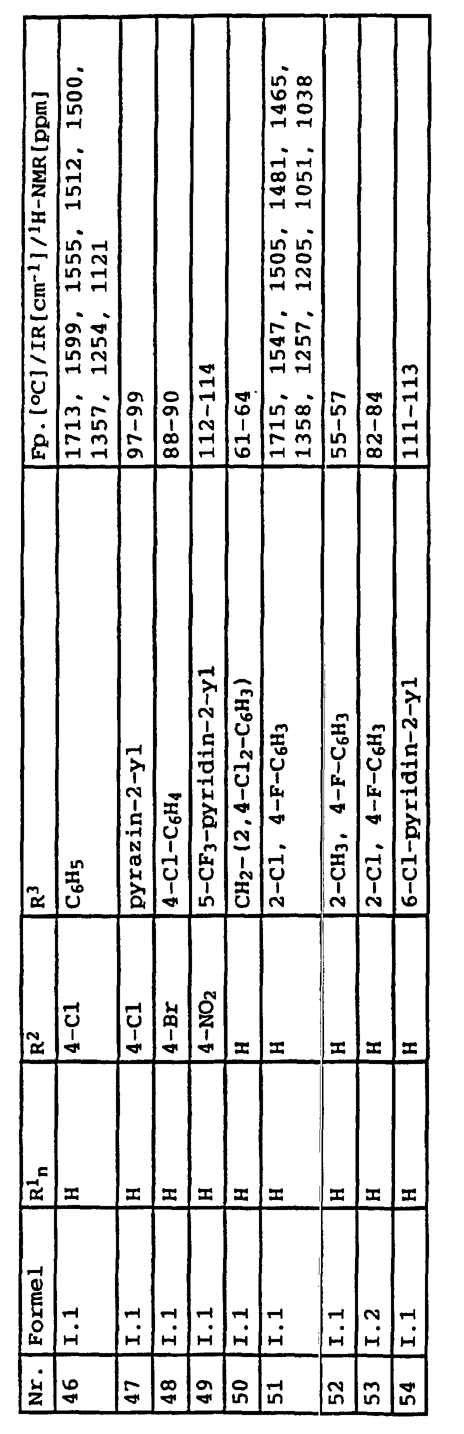

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

Als Vergleichsverbindungen dienten die folgenden, aus EP-A 513 580 bekannten Wirkstoffe:
A Beispiel Nr. 17 aus EP-A 513 580
B Beispiel Nr. 56 aus EP-A 513 580
C Beispiel Nr. 57 aus EP-A 513 580
D Beispiel Nr. 143 aus EP-A 513 580
E Beispiel Nr. 144 aus EP-A 513 580
F Beispiel Nr. 145 aus EP-A 513 580

### 1. Erysiphe graminis var. tritici

Blätter von Weizenkeimlingen (Sorte "Frühgold") wurden zunächst mit der wäßrigen Aufbereitung der Wirkstoffe (16 ppm-haltige Aufbereitung) behandelt. Nach ca. 24 h wurden die Pflanzen mit Sporen des Weizenmehltaus (Erysiphe graminis var. tritici) bestäubt. Die so behandelten Pflanzen wurden anschließend für 7 Tage bei 20-22°C und einer relativen Luftfeuchtigkeit von 75-80% inkubiert. Anschließend wurde das Ausmaß der Pilzentwicklung ermittlelt. Die Auswertung erfolgte visuell.

In diesem Test zeigten die mit den erfindungsgemäßen Verbindungen 1, 2, 3, 4, 5, 6, 10 12, 13, 15, 16 und 17 behandelten Pflanzen einen Befall von 15% und weniger, während die mit den bekannten Wirkstoffen behandelten Pflanzen zu 70% (A und C), 60% (B) bzw. 30% (D, E, und F) befallen waren. Der Befall bei den nichtbehandelten Kontrollpflanzen war 70%.

### 2. Plasmopara viticola

Topfreben (Sorte: "Müller Thurgau") wurden mit der Wirkstoffaufbereitung tropfnaß gespritzt. Nach 8 Tagen wurden die Pflanzen mit einer Zoosporenaufschwemmung des Pilzes Plasmopara viticola besprüht und 5 Tage bei 20-30 °C bei hoher Luftfeuchtigkeit bewahrt. Vor der Beurteilung wurden die Pflanzen danach für 16 h bei hoher Luftfeuchtigkeit bewahrt.

Die Auswertung erfolgte visuell.

In diesem Test zeigten die mit 63 ppm haltigen Aufbereitungen der erfindungsgemäßen Verbindungen 1, 2, 4, 5, 6, 7, 8, 9, 12, 13, 14 und 18 behandelten Pflanzen einen Befall von 5% und weniger, während die mit 125 ppm haltigen Aufbereitungen der bekannten Wirkstoffe behandelten Pflanzen zu 60% (A und F), 40%, (C) bzw. 25% (E) befallen waren. Der Befall der nichtbehandelten Kontrollpflanzen war 60%.

## Patentansprüche

1. α-Phenylbutensäuremethylester der Formel I 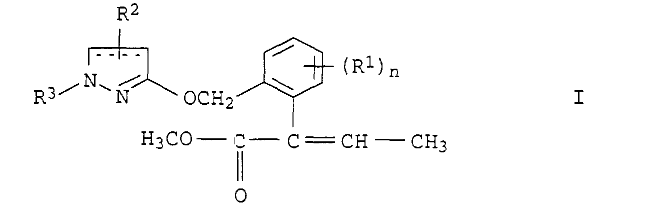 in der -- für eine Einfach- oder Doppelbindung steht und der Index und die Substituenten die folgende Bedeutung haben:
n 0, 1, 2, 3 oder 4, wobei die Substituenten R¹ verschieden sein können, wenn n größer als 1 ist;
R¹ Nitro, Cyano, Halogen, C₁-C₄-Alkyl, C₁-C₄-Halogenalkyl oder C₁-C₄-Alkoxy;
R² Wasserstoff, Nitro, Cyano, Halogen, C₁-C₄-Alkyl, C₁-C₄-Halogenalkyl, C₁-C₄-Alkoxy, C₁-C₄-Alkylthio oder C₁-C₄-Alkoxycarbonyl;
R³ C₁-C₁₀-Alkyl, C₂-C₁₀-Alkenyl oder C₂-C₂₀-Alkinyl; wobei diese Gruppen partiell oder vollständig halogeniert sein können und/oder einen bis drei der folgenden Reste tragen können:
Cyano, Nitro, C₁-C₄-Alkoxy, C₁-C₄-Halalkoxy, C₁-C₄-Alkylthio oder Phenyl und Naphthyl und 5-Ring Heteroaromaten enthaltend ein bis drei Stickstoffatome und/ oder ein Sauerstoff- oder Schwefelatom oder Sechsring
Heteroatomaten enthaltend ein bis vier Stickstoffatome,
wobei die cyclischen Gruppen partiell oder vollständig halogeniert sein können und/oder einen bis drei der folgenden Reste tragen können: Nitro, Cyano, C₁-C₄-Alkyl oder C₁-C₄-Alkoxy;
ein gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, oder ein ein- oder zweikerniger aromatischer Rest, welcher neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann,
wobei die cyclischen Gruppen partiell oder vollständig halogeniert sein können und/oder einen bis drei der folgenden Reste tragen können: Nitro, Cyano, C₁-C₄-Alkyl oder C₁-C₄-Alkoxy.

2. Verfahren zur Herstellung der Verbindungen der Formel I gemäß Anspruch 1, **dadurch gekennzeichnet**, daß man ein Benzylderivat der Formel II, 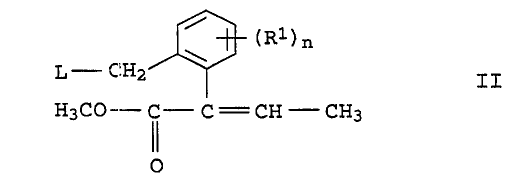 in der L eine nucleophil austauschbare Gruppe bedeutet, in Gegenwart einer Base mit einem 3-Hydroxy(dihydro)pyrazol der Formel III 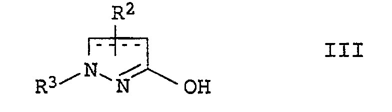 umsetzt.

3. Zur Bekämpfung von Schadpilzen geeignete Mischung, enthaltend einen inerten Zusatzstoff und eine wirksame Menge einer Verbindung der Formel I gemäß Anspruch 1.

4. Zur Bekämpfung von tierischen Schädlingen geeignete Mischung, enthaltend einen inerten Zusatzstoff und eine wirksame Menge einer Verbindung der Formel I gemäß Anspruch 1.

5. Verfahren zur Bekämpfung von Schadpilzen, **dadurch gekennzeichnet**, daß man die Schadpilze, ausgenommen am tierischen Körper, ihren Lebensraum oder die von Schadpilzen freizuhaltenden Pflanzen, Flächen, Materialien oder Räume mit einer wirksamen Menge einer Verbindung der Formel I gemäß Anspruch 1 behandelt.

6. Verfahren zur Bekämpfung von tierischen Schädlingen, **dadurch gekennzeichnet**, daß man die Schädlingen, ausgenommen am tierischen Körper, ihren Lebensraum oder die von Schädlingen freizuhaltenden Pflanzen, Flächen, Materialien oder Räume mit einer wirksamen Menge einer Verbindung der Formel I gemäß Anspruch 1 behandelt.

7. Verwendung der Verbindungen I gemäß Anspruch 1 zur Herstellung einer zur Bekämpfung von Schadpilzen geeigneten Mischung.

8. Verwendung der Verbindungen I gemäß Anspruch 1 zur Herstellung einer zur Bekämpfung von tierischen Schädlingen geeigneten Mischung.

9. Verwendung der Verbindungen I gemäß Anspruch 1 zur Bekämpfung von Schadpilzen, ausgenommen am tierischen Körper.

10. Verwendung der Verbindungen I gemäß Anspruch 1 zur Bekämpfung von tierischen Schädlingen, ausgenommen am tierischen Körper.

## Claims

1. A methyl α-phenylbutenoate of the formula I 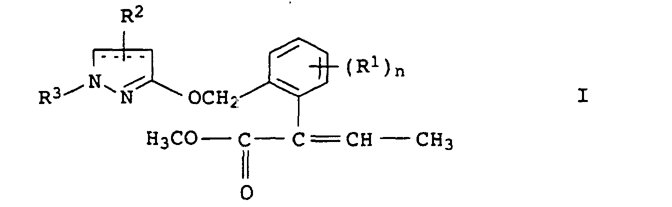 where -- is a single or double bond and the index and the substituents have the following meanings:
n is 0, 1, 2, 3 or 4, it being possible for the substituents R¹ to be different if n is greater than 1;
R¹ is nitro, cyano, halogen, C₁-C₄-alkyl, C₁-C₄-haloalkyl or C₁-C₄-alkoxy;
R² is hydrogen, nitro, cyano, halogen, C₁-C₄-alkyl, C₁-C₄-haloalkyl, C₁-C₄-alkoxy, C₁-C₄-alkylthio or C₁-C₄-alkoxycarbonyl;
R³ is C₁-C₁₀-alkyl, C₂-C₁₀-alkenyl or C₂-C₂₀-alkynyl; where these groups can be partly or completely halogenated and/or can carry one to three of the following radicals:
cyano, nitro, C₁-C₄-alkoxy, C₁-C₄-haloalkoxy, C₁-C₄-alkylthio or phenyl and naphthyl and 5-membered ring heteroaromatics containing one to three nitrogen atoms and/or one oxygen or sulfur atom or six-membered ring heteroaromatics containing one to four nitrogen atoms,
a saturated or mono- or diunsaturated ring which, in addition to carbon atoms, can contain one to three of the following heteroatoms as ring members: oxygen, sulfur and nitrogen, or
a mono- or binuclear aromatic radical which, in addition to carbon atoms, can contain one to four nitrogen atoms or one or two nitrogen atoms and an oxygen or sulfur atom or an oxygen or sulfur atom as ring members,
where the cyclic groups can be partly or completely halogenated and/or can carry one to three of the following radicals: nitro, cyano, C₁-C₄-alkyl or C₁-C₄-alkoxy;

2. A process for preparing the compounds of the formula I as claimed in claim 1, which comprises reacting a benzyl derivative of the formula II 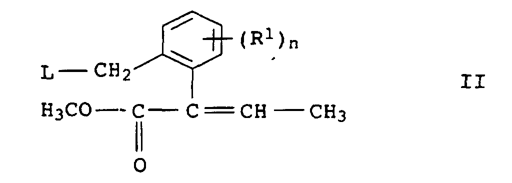 where L is a nucleophilically replaceable group, in the presence of a base with a 3-hydroxy(dihydro)pyrazole of the formula III

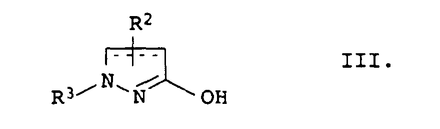

3. A mixture suitable for controlling harmful fungi, containing an inert additive and an active amount of a compound of the formula I as claimed in claim 1.

4. A mixture suitable for controlling animal pests, containing an inert additive and an active amount of a compound of the formula I as claimed in claim 1.

5. A method of controlling harmful fungi, which comprises treating the harmful fungi, except on animals' bodies, their habitat or the plants, surfaces, materials or spaces to be kept free from them with an active amount of a compound of the formula I as claimed in claim 1.

6. A method of controlling animal pests, which comprises treating the pests, except on animals' bodies, their habitat or the plants, surfaces, materials or spaces to be kept free from them with an active amount of a compound of the formula I as claimed in claim 1.

7. The use of the compounds I as claimed in claim 1 for the production of a mixture suitable for controlling harmful fungi.

8. The use of the compounds I as claimed in claim 1 for the production of a mixture suitable for controlling animal pests.

9. The use of the compounds I as claimed in claim 1 for controlling harmful fungi, except on animals' bodies.

10. The use of the compounds I as claimed in claim 1 for controlling animal pests, except on animals' bodies.

## Revendications

1. Alpha-phénylbuténoates de méthyle de formule I 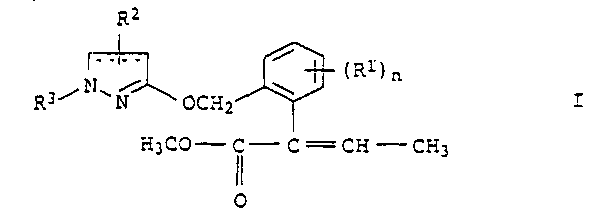 dans laquelle représente une liaison simple ou double, et l'indice et les symboles ont les significations suivantes :
n 0, 1, 2, 3 ou 4, les substituants R¹ pouvant être différents lorsque n est supérieur à 1 ;
R¹ un groupe nitro, cyano, un halogène, un groupe alkyle en C1-C4, halogénoalkyle en C1-C4 ou alcoxy en C1-C4 ;
R² l'hydrogène, un groupe nitro, cyano, un halogène, un groupe alkyle en C1-C4, halogénoalkyle en C1-C4, alcoxy en C1-C4, alkylthio en C1-C4 ou (alcoxy en C1-C4)carbonyle ;
R³ un groupe alkyle en C1-C10, alcényle en C2-C10 ou alcynyle en C2-C20;
ces groupes pouvant être halogénés en totalité ou en partie et/ou pouvant porter un à trois des substituants suivants :
cyano, nitro, alcoxy en C1-C4, halogénoalcoxy en C1-C4, alkylthio en C1-C4 ou phényle, naphtyle ou groupe hétéroaromatique à cinq chaînons contenant un à trois atomes d'azote et/ou un atome d'oxygène ou de soufre ou groupe hétéroaromatique à six chaînons contenant un à quatre atomes d'azote,
les groupes cycliques pouvant être halogénés en totalité ou en partie et/ou pouvant porter un à trois des substituants suivants : nitro, cyano, alkyle en C1-C4 ou alcoxy en C1-C4;
un cycle saturé ou mono- ou di-insaturé qui, en plus des atomes de carbone, peut contenir un à trois des hétéroatomes suivants en tant que chaînons cycliques : oxygène, soufre et azote, ou bien,
un radical aromatique mono- ou bi-cyclique qui, en plus des atomes de carbone, peut contenir un à quatre atomes d'azote ou un ou deux atomes d'azote et un atome d'oxygène ou de soufre ou un atome d'oxygène ou de soufre en tant que chaînons cycliques,
les groupes cycliques pouvant être halogénés en totalité ou en partie et/ou pouvant porter un à trois des substituants suivants : nitro, cyano, alkyle en C1-C4 ou alcoxy en C1-C4.

2. Procédé de préparation des composés de formule I de la revendication 1, **caractérisé en ce que** l'on fait réagir un dérivé benzylique de formule II 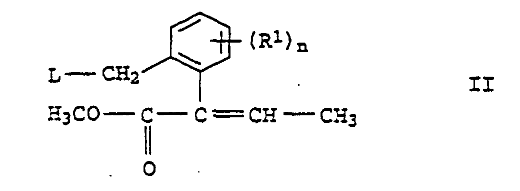 dans laquelle L représente un groupe apte à échange nucléophile, en présence d'une base, avec un 3-hydroxy(dihydro)pyrazole de formule III

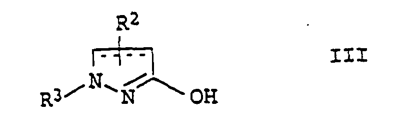

3. Mélange approprié à l'utilisation pour la lutte contre les mycètes nuisibles, contenant un additif inerte et une quantité efficace d'un composé de formule I de la revendication 1.

4. Mélange approprié à l'utilisation pour la lutte contre les parasites animaux, contenant un additif inerte et une quantité efficace d'un composé de formule I de la revendication 1.

5. Procédé pour combattre les mycètes nuisibles, **caractérisé en ce que** l'on traite les mycètes nuisibles, mais non sur les corps d'animaux, leur habitat ou les végétaux, aires, matériaux ou locaux qu'on veut protéger contre les mycètes nuisibles par une quantité efficace d'un composé de formule I de la revendication 1.

6. Procédé pour combattre les parasites animaux, **caractérisé en ce que** l'on traite les parasites, mais non sur les corps d'animaux, leur habitat ou les végétaux, aires, matériaux ou locaux qu'on veut protéger contre les parasites animaux par une quantité efficace d'un composé de formule I de la revendication 1.

7. Utilisation des composés I de la revendication 1 pour la préparation d'un mélange approprié à l'utilisation pour la lutte contre les mycètes nuisibles.

8. Utilisation des composés I de la revendication 1, pour la préparation d'un mélange approprié à l'utilisation pour la lutte contre les parasites animaux.

9. Utilisation des composés I de la revendication 1, pour la lutte contre les mycètes nuisibles, mais non sur les corps des animaux.

10. Utilisation des composés I selon revendication 1, pour la lutte contre les parasites animaux, sauf sur les corps d'animaux.
